Europäisches Patentamt

European Patent Office

Office européen des brevets

(11) Veröffentlichungsnummer: **0 354 485**
**A2**

(12) **EUROPÄISCHE PATENTANMELDUNG**

(21) Anmeldenummer: 89114398.4

(51) Int. Cl.⁴: **C12N 11/10**

(22) Anmeldetag: 03.08.89

(30) Priorität: 09.08.88 DE 3827001

(43) Veröffentlichungstag der Anmeldung:
14.02.90 Patentblatt 90/07

(84) Benannte Vertragsstaaten:
**AT BE CH DE ES FR GB GR IT LI LU NL SE**

(71) Anmelder: **Hüttermann, Alois Prof. Dr.**
**Forstbotanisches Institut Büsgenweg 2**
**D-3400 Göttingen(DE)**

(72) Erfinder: **Hüttermann, Aloys, Prof. Dr.**
**Büsgenweg 2**
**D-3400 Göttingen(DE)**
Erfinder: **Milsteinn, Oleg, Dr.**
**Ludwig-Beck-Strasse 11**
**D-3400 Göttingen(DE)**
Erfinder: **Nicklas, Barbara, Dipl.-Forstw.**
**Bismarckstrasse 120**
**D-3400 Göttingen(DE)**

(74) Vertreter: **Harders, Gerhard, Dr.**
**Stettiner Strasse 2**
**D-6367 Karben 6(DE)**

(54) **Enzym-Matrix-Komplexe, Verfahren zu ihrer Herstellung und ihre Verwendung.**

(57) Ligninmodifizierende Enzyme lassen sich in eine Matrix auf Polysaccharidbasis einbetten und bilden dabei Enzym-Matrix-Komplexe, die die Anwendung der ligninmodifizierenden Enzyme in organischen Lösungsmitteln ermöglichen.

EP 0 354 485 A2

## Enzym-Matrix-Komplexe, Verfahren zu ihrer Herstellung und ihre Verwendung

Die vorliegende Erfindung betrifft Enzym-Matrix-Komplexe, die aus ligninmodifizierenden Enzymen bestehen, die in eine Matrix auf Polysaccharidbasis eingebettet sind, sowie Verfahren zu deren Herstellung und ihre Verwendung für Enzymreaktionen in hydrophilen und hydrophoben organischen Lösungsmitteln. Die Erfindung ermöglicht erstmalig die Anwendung ligninmodifizierender Enzyme in organischen Lösungsmitteln und ermöglicht damit u. a. die Herstellung polymerer Produkte von Lignin bzw. Ligninderivaten.

Die Herstellung des phenoloxidierenden Enzyms Laccase ist beispielsweise in der DE-PS 30 37 992 beschrieben. Bei diesem Herstellungsverfahren fällt das Enzym in wässriger Lösung an. Auch die weiteren Reaktionen des Enzyms werden in wässrigen Medien durchgeführt. Umsetzungen des Enzyms in organischen Lösungsmitteln sind in dieser Druckschrift nicht erwähnt.

Aufgabe der vorliegenden Erfindung ist es, neue Enzym-Matrix-Komplexe von ligninmodifizierenden Enzymen zu schaffen, die ihre Enzymaktivität auch in organischen Lösungsmitteln weitgehend beibehalten und damit beispielsweise die Umsetzung von Lignin bzw. Ligninderivaten in organischen Lösungsmitteln ermöglichen.

Gelöst wird diese Aufgabe durch die im Hauptanspruch gekennzeichneten Enzym-Matrix-Komplexe.

Als Bestandteile der erfindungsgemäßen Enzym-Matrix-Komplexe eignen sich alle ligninmodifizierenden Enzyme. Hierunter fallen Oxidasen, wie beispielsweise Laccasen, Lignin-Peroxidasen, Mn-Peroxidasen oder Poly-Blue-Oxidasen.

Als Matrix-bildende Polysaccharide können beispielsweise alle Polysaccharide verwendet werden, die ein poröses, inertes, lösungsmittelfestes Matrixgel bilden. Unter ihnen sind Agarosegele und Dextrangele sowie deren Derivate und Vernetzungsprodukte besonders bevorzugt. Unter den Derivaten sind z. B. Alkyl- oder Hydroxyalkylderivate, deren Alkylgruppe 1 bis 3 bzw. bis 4 Kohlenstoffatome enthalten kann. Auch Vernetzungsprodukte mit anderen organischen Stoffen, die eine Vernetzung bewirken können, wie z. B. N,N'-Methylenbis(acrylamid) sind als Matrixgele geeignet.

Die verwendeten Matrixgele sollen bevorzugt eine Ausschlußgrenze in einem Molgewichtsbereich von etwa 30.000 bis etwa 1.000.000 aufweisen, da die üblichen ligninmodifizierenden Enzyme bevorzugt in solche Matrixgele eingebettet werden können.

Die erfindungsgemäßen Enzym-Matrix-Komplexe sind in nahezu allen organischen Lösungsmitteln wirksam, sowohl in hydrophilen als auch in hydrophoben Lösungsmitteln. Von der Verwendung sind lediglich solche Lösungsmittel ausgeschlossen, die mit Metallen Chelate bilden, da beispielsweise die Laccasen Kupfer enthalten. Insbesondere lassen sich die erfindungsgemäßen Enzym-Matrix-Komplexe in den folgenden organischen Lösungsmitteln verwenden:

Ethylacetat, Benzol, Toluol, Ether, Isooctan, n-Hexan, Cyclohexan, Chloroform, Dichlorethan, Acetonitril, Aceton, Methanol, Ethanol, 1,4-Dioxan, Tetrahydrofuran u.a.

Beim Einsatz der erfindungsgemäßen Enzym-Matrix-Komplexe für Enzymreaktionen in organischen Lösungsmitteln ist die Gegenwart zumindest kleiner Mengen an Wasser erforderlich. Deshalb werden bei der Verwendung in hydrophilen organischen Lösungsmittel etwa 1 bis 80 Vol.-% Wasser, vorzugsweise etwa 3,5 bis 50 Vol.-%, bezogen auf die Lösungsmittelmenge, hinzugegeben. Beim Einsatz von Hydrphoben organischen Lösungsmitteln werden diese mit Wasser gesättigt.

Überraschenderweise zeigen die erfindungsgemäßen Enzym-Matrix-Komplexe auch in organischen Lösungsmitteln einen hohen Aktivitätsgrad, der unabhängig ist vom hydrophilen Charakter der Lösungsmittels. Sie haben eine hohe Stabilität, auch bei höheren temperaturen, und hohe Toleranz gegenüber Enzymgiften, wie Natriumazid und Wasserstoffperoxid.

Die erfindungsgemäßen Enzym-Matrix-Komplexe ermöglichen den biologischen Abbau von durch ligninmodifizierende Enzyme abbaubaren Stoffen, sowie biologische Oxidations- und Polymerisationsreaktionen, die durch solche Enzyme bewirkt werden. So können beispielsweise aus Lignin oder Ligninderivaten hochreaktive Lignine hergestellt werden. Als reaktive Gruppen können dabei oxidierte Gruppen im Ligninmolekül erhalten werden. Auf diese Weise ist es möglich, Ligninprepolymere zu erhalten, die bei der Herstellung von Kunststoffen Verwendung finden können.

Die Herstellung der erfindungsgemäßen Enzym-Matrix-Komplexe kann in einfacher Weise durch Vermischen von Lösungen der Enzyme mit der betreffenden Matrixsubstanz erfolgen. Dabei wird das Enzym in einer Pufferlösung der folgenden Zusammensetzung gelöst:

Citratpuffer:

Eine wässrige 0,1 M Lösung von Citronensäure wird mit einer wässrigen 0,2 M Lösung von $K_2HPO_4$ auf einen pH-Wert von 4,5 eingestellt.

Die Herstellung des Komplexes kann aber auch durch Aufbringen der gepufferten Enzymlösung auf eine mit dem Matrixgel gefüllte Chromatographie-Säule erfolgen. Das den Enzym-Matrix-Komplex enthaltende Matrixgel wird gewonnen und lyophilisiert (gefriergetrocknet).

Die Erfindung wird nachfolgend anhand der Beispiele näher erläutert, wobei alle in den Beispielen enthaltenen Merkmale als erfindungswesentlich angesehen werden.

Beispiel 1

Herstellung eines Laccase-Dextrangel-Komplexes

Eine 1,9 x 9 cm Chromatographie-Säule wurde mit einer wässrigen Suspension eines Dextrangels vom Typ Sepharose CL 6 B (1,3 g Trockengewicht) gepackt und die Füllung mit 0,1 M Citratpuffer (pH-Wert 4,5) äquilibriert. Das Ausschlußvolumen dieser Säule betrug 7 ml.

7,5 ml einer Lösung von Laccase in Citratpuffer, enthaltend 135 mg Laccase-Protein, wurden auf die Säule gegeben und in die Säule einlaufen gelassen. Nach dem Einlauf der Lösung wurde das Laccase-haltige Gelbett aus der Säule entnommen und bei -80° eingefroren. Das gefrorene Gel wurde dann lyophilisiert und ergab frei fließende Gelkügelchen, die die Laccase enthielten. Der Gehalt des Enzym-Matrix-Komplexes an Laccase betrug im Mittel 104 µg Enzymprotein pro 1 mg Gel.

Beispiel 2

Reaktion des nach Beispiel 1 erhaltenen Enzym-Matrix-Komplexes mit den Substraten 2,6-Dimethoxyphenol und Syringaldazin

5,5 mg des nach Beispiel 1 erhaltenen Komplexes wurden in 10 ml Ethylacetat, das mit Wasser gesättigt war, suspendiert. Davon wurden 100 µl in eine 3 ml Küvette (Lichtpaß 10 mm) gegeben und 2,5 ml Substrat in mit Wasser gesättigtem Ethylacetat zugegeben. Die wirksame Enzymmenge betrug 5,7 µg Enzymprotein.

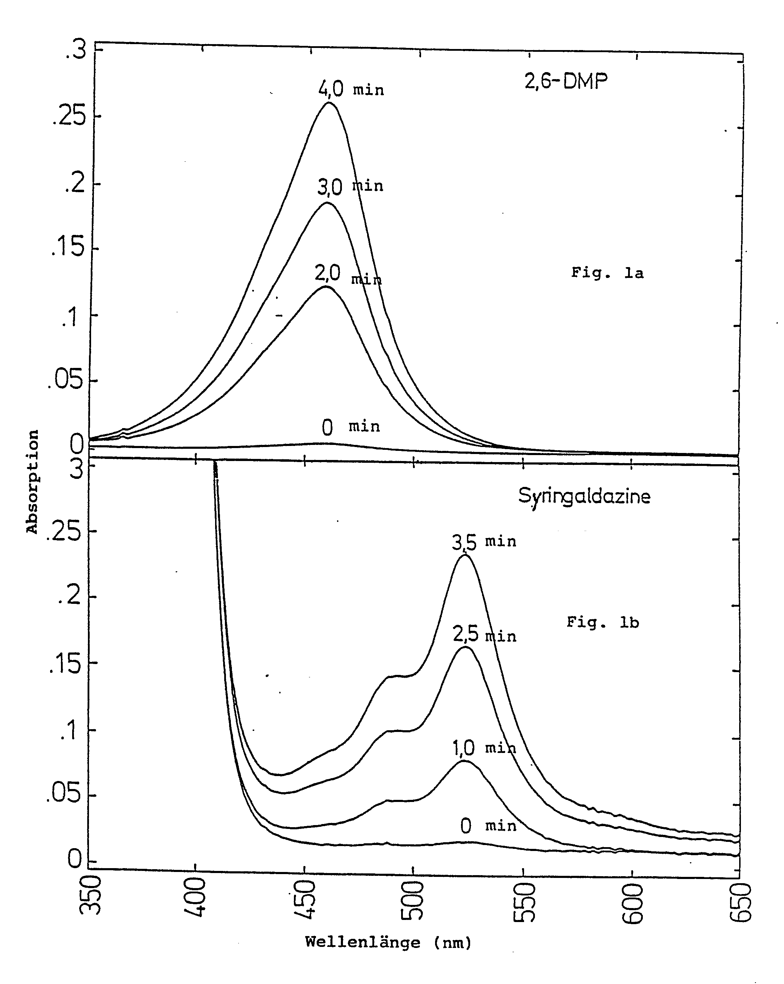

Die Küvetten wurden unter Rühren bei 30° thermostatisiert. Infolge der Oxidation von DMP bzw. Syringaldazin färbte sich die Lösung schnell gelb bzw. rotviolett. Die Figuren 1a und 1b zeigen die Änderung der Absorption infolge der Oxidation der beiden Substrate durch den Enzym-Matrix-Komplex. Die Reaktionsgeschwindigkeit wird dargestellt in Form der Änderungder Absorption bei 458 nm pro Minute bei einer eingesetzten Enzymmenge von 5,7 ug (für DMP) und Syringaldazin (55 µg Komplexe enthalten5,7 ug Enzym). Die Reaktionsgeschwindigkeit ist somit definiert als
458 nm . $min^{-1}$ mg $Enzym^{-1}$

Die Messungen wurden mit den folgenden Lösungsmitteln wiederholt, die jeweils 7,0 Vol.-% Wasser enthielten:
Acetonitril, Aceton und Tetrahydrofuran.

Die Ergebnisse sind in der Tabelle 1 zusammengestellt.

Entsprechende Messungen wurden mit Syringaldazin durchgeführt. Die Änderung der Absorption bei 520 nm in Abhängigkeit von der Zeit ist in Figur 1b dargestellt. Die Reaktionsgeschwindigkeit für Syringaldehyd ist definiert als µmol oxidiertes Syringaldazin-Substrat pro Minute pro mg Enzym. Gemäß J. M. Harkin und J. R. Obst (Experientia Vol. 29, S. 381-7, 1973) entspricht eine Änderung der Absorption um 0,065 einer Menge von 1 µmol oxidiertem Produkt. Die Reaktionsgeschwindigkeit ist demnach definiert als
1 µmol . $min^{-1}$ . mg $Enzym^{-1}$ Die Ergebnisse sind in Tabelle 1 zusammengestellt.

Tabelle 1

| Substrat | Lösungsmittel | Reaktionsgeschwindigkeit | |
|---|---|---|---|
| 2,6-DMP | Ethylacetat[a] | 11,6 | 468 nm . $\text{min}^{-1}$ . mg $\text{E}^{-1}$ |
| | Acetonitril[b] | 12,6 | " |
| | Aceton[b] | 21,3 | " |
| | Tetrahydrofuran[b] | 3,4 | " |
| Syringaldazin | Ethylacetat[a] | 216 | $\mu$mol • $\text{min}^{-1}$ . mg $\text{E}^{-1}$ |
| | Acetonitril[b] | 282 | " |
| | Aceton[b] | 206 | " |
| | Tetrahydrofuran | 36 | " |

[a] Ethylacetat, gesättigt mit Wasser
[b] Lösungsmittel, enthaltend 7,0 Vol.-% Wasser

Beispiel 3

Umsetzung von Syringaldazin in verschiedenen Lösungsmitteln

Entsprechend Beispiel 2 wurde Syringaldazin in verschiedenen Lösungsmitteln mit dem nach Beispiel 1 erhaltenen Enzym-Matrix-Komplex umgesetzt. Die dabei erhaltenen Ergebnisse sind in Tabelle 2 zusammengestellt.

Tabelle 2

| Lösungsmittel | Reaktionsgeschwindigkeit umol\|min\|mg Enzym |
|---|---|
| mit Wasser gesättigtes | |
| Ethylacetet | 216 |
| Toluol | 104 |
| Benzol | 81 |
| Ether | 75 |
| Isooctan | 68 |
| n-Hexan | 62 |
| Cyclohexan | 48 |
| Chloroform | 15 |
| Dichlorethan | 8 |
| Lösungsmittel + 3,5 Vol.-% Wasser: | |
| Acetonitril | 116 |
| Aceton | 80 |
| Ethanol | 28 |
| 1,4-Dioxan | 16 |
| Tetrahydrofuran | 11 |
| Methanol | 10 |

Beispiel 4

Umsetzung von Lignin mit Enzym-Matrix-Komplex

Als Substrat wurde synthetisches Lignin aus Coniferylalkohol (DHP) verwendet, dessen -OCH₃-Gruppen mit radioaktivem ¹⁴C markiert waren. Radioaktivität von 1 mg = 615.570 dpm.

Die Reaktion wurde in einem mit einem Gummideckel verschlossenen Erlenmeyerkolben durchgeführt, in dem sich ein von der Bodenflüssigkeit getrenntes Absorptionsgefäß befand, das mit 1 ml Natronlauge gefüllt war.

In dem Erlenmeyerkolben wurden 1 mg DHP * in 10 ml Ethylacetat (mit Wasser gesättigt) mit 15 mg Enzym-Matrix-Komplex nach Beispiel 1 bei 25° vorsichtig geschüttelt. Die Natronlauge im Absorptionsgefäß wurde täglich gewechselt. Die Radioaktivität in den jeweiligen Natronlauge-Proben wurde bestimmt nach Haider und Trojanowski, Arch. Microbiol. 105, 33-41, 1975.

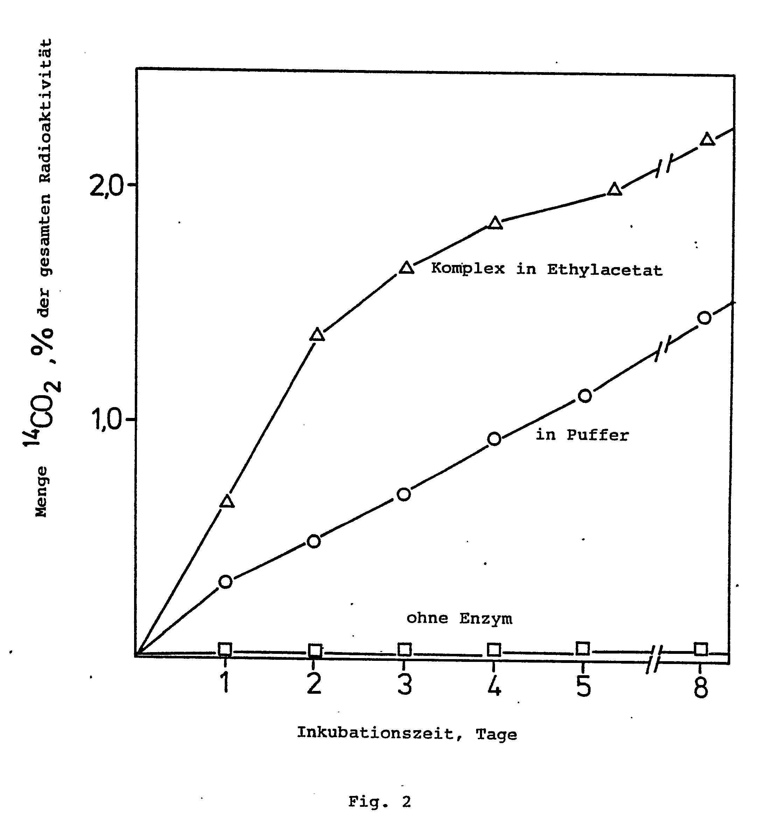

Als Kontrolle wurden jeweils Messungen durchgeführt, in denen Ethylacetat durch Citratpuffer ersetzt worden war. Die Ergebnisse sind in der Figur 2 dargestellt. Ein weiterer Kontrollversuch wurde ohne aktives Enzym (mit Ethylacetat und DHP thermisch inaktiviert) durchgeführt.

Die Aktivität des Enzym-Matrix-Komplexes beträgt in organischen Lösungsmitteln mehr als das Doppelte des Wertes in Citratpuffer.

Beispiel 5

150 mg Organosolv-Lignin wurden in 3 ml Dioxan, enthaltend 20 Vol.-% Wasser, gelöst, die Lösung zentrifugiert und der Überstand mit 15 mg des nach Beispiel 1 erhaltenen Enzym-Matrix-Komplexes versetzt. Das Gemisch wurde 3 tage bei 25° im Dunkeln geschüttelt. Dann wurde das gemisch zentrifugiert und die klare Lösung einer HPSEC-Chromatographie unterworfen, wie in Wat. Sci. Techn., Vol. 20, S. 161.170, 1988 beschrieben ist.

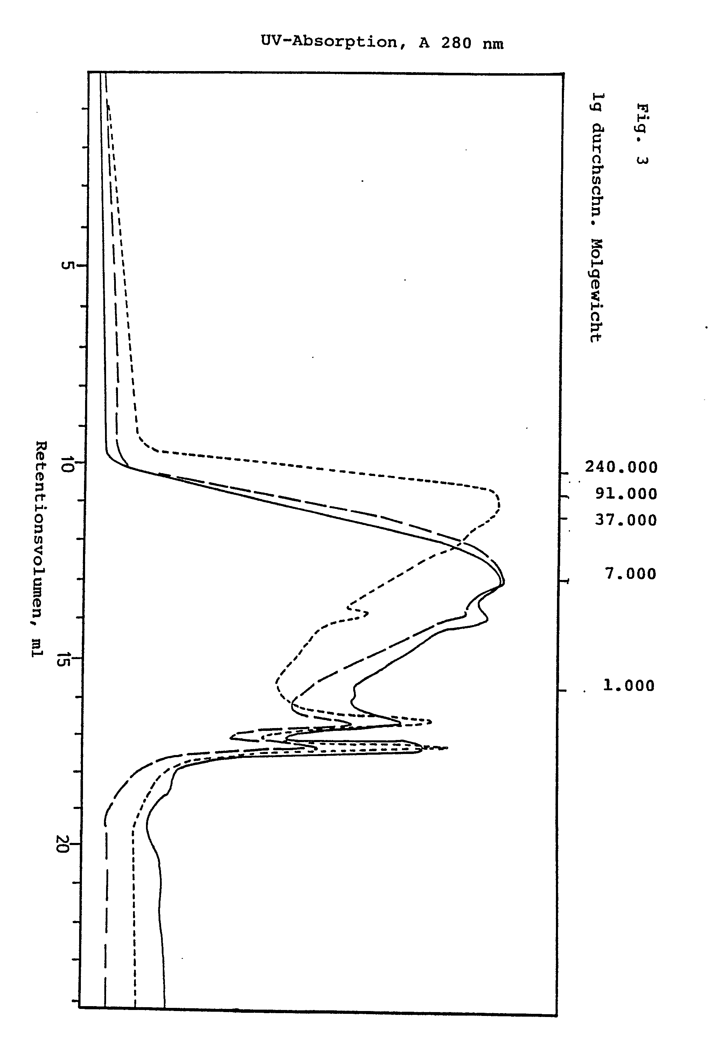

Das Ergebnis ist in Fig. 3 dargestellt. Das Ausgangsprodukt hatte ein Molekulargewicht von ca. 7000. Das Molekulargewicht war nach 3tägigem Schütteln ohne Enzym nahezu unverändert. Nach der Enzymbehandlung erhöhte sich das Molekulargewicht auf ca. 240.000.

Die Ergebnisse der Beispiele 4 und 5 zeigen, daß durch die Behandlung mit dem Enzym-Matrix-Komplex bei Lignin nicht reaktive -OCH₃-Gruppen in hochreaktive -OH-Gruppen umgesetzt werden und daß außerdem aus dem Lignin höhermolekulare Prepolymere erhalten werden können. Diese reaktiven Prepolymere eignen sich zum Einsatz in der Kunststoffherstellung. Sie haben gegenüber herkömmlichen Kunststoffen den Vorteil, biologisch abbaubar zu sein.

**Ansprüche**

1. Enzym-Matrix-Komplexe, bestehend aus ligninmodifizierenden Enzymen, die in einer Matrix auf Polysaccharidbasis eingebettet sind.

2. Enzym-Matrix-Komplex nach Anspruch 1, dadurch gekennzeichnet, daß die ligninmodifizierenden Enzyme aus Oxidasen, wie Laccasen, Lignin-Peroxidasen, Mn-Peroxidasen oder Poly-Blue-Oxidasen bestehen.

3. Enzym-Matrix-Komplex nach Anspruch 1, dadurch gekennzeichnet, daß die Matrix eine poröse, inerte, lösungsmittelfeste Matrix auf Polysaccharidbasis ist.

4. Enzym-Matrix-Komplex nach Anspruch 1, dadurch gekennzeichnet, daß die Matrix aus den folgenden Polysacchariden besteht: Agarosegele, Dextrangele und dergl. sowie deren Derivate und Vernetzungsprodukte.

5. Enzym-Matrix-Komplex nach Anspruch 1, dadurch gekennzeichnet, daß die Matrixgele Ausschlußgrenzen von etwa 30.000 bis etwa 1.000.000 aufweisen.

6. Verfahren zur Herstellung der Enzym-Matrix-Komplexe nach den Ansprüchen 1 bis 5, dadurch gekennzeichnet, daß die Enzyme in einer Pufferlösung gelöst und mit den Matrixgelen vermischt werden.

7. Verfahren nach Anspruch 6, dadurch gekennzeichnet, daß das Vermischen durch Aufgeben der Enzymlösung auf eine mit dem Matrixgel gefüllte Chromatographie-Säule erfolgt.

8. Verwendung der Enzym-Matrix-Komplexe nach den Ansprüchen 1 bis 5 für Enzymreaktionen in hydrophilen und hydrophoben organischen Lösungsmitteln, wobei in hydrophilen organischen Lösungsmitteln 1 bis 80 Vol.-% Wasser hinzugegeben werden bzw. in hydrophoben Organischen Lösungsmitteln die

* DHP = synthetisches Lignin

Lösungsmittel mit Wasser gesättigt werden.

9. Verwendung nach Anspruch 8, wobei in hydrophilen organischen Lösungsmitteln etwa 3,5 bis 50 Vol.-% Wasser hinzugegeben werden.

10. Verfahren zur Herstellung von polymeren Produkten von Lignin bzw. Ligninderivaten in organischen Lösungsmitteln unter verwendung der Enzym-Matrix-Komplexe nach den Ansprüchen 1 bis 5.

Fig. 1a

Fig. 1b

Fig. 2

Fig. 3